Europäisches Patentamt

European Patent Office

Office européen des brevets

(11) Publication number: **0 361 905**
**A2**

## EUROPEAN PATENT APPLICATION

(21) Application number: 89309855.8

(51) Int. Cl.⁵: **C12N  15/52** , **C12N  15/76**

(22) Date of filing: **27.09.89**

Claims for the following Contracting States: ES + GR

(30) Priority: **29.09.88 US 251158**

(43) Date of publication of application: .
**04.04.90 Bulletin  90/14**

(84) Designated Contracting States:
**AT BE CH DE ES FR GB GR IT LI NL SE**

(71) Applicant: **ELI LILLY AND COMPANY**
**Lilly Corporate Center**
**Indianapolis Indiana 46285(US)**

(72) Inventor: **Epp, Janet Kay**
**920 North Graham Avenue**
**Indianapolis Indiana 46219(US)**
Inventor: **Schoner, Brigitte Elisabeth**
**R.R. 2, Box 30 F**
**Monrovia Indiana 46157(US)**

(74) Representative: **Hudson, Christopher Mark et al**
**Erl Wood Manor**
**Windlesham Surrey GU20 6PH(GB)**

(54) **Carbomycin biosynthetic genes, designated carL and carM, for use in streptomyces and other organisms.**

(57) The carL and carM genes of Streptomyces thermotolerams have been isolated and used to construct recombinant DNA expression vectors. The carL and carM encode activities required for the biosynthesis of macrolide antibiotics. These genes have been shown to complement Streptomyces fradiae mutants which lack the ability to produce the antibiotic, tylosin. The carL and carM genes can be used not only to construct recombinant cells with increased ability to produce macrolides but also to construct recombinant cells with the ability to produce novel antibiotic compounds.

EP 0 361 905 A2

# CARBOMYCIN BIOSYNTHETIC GENES, DESIGNATED carL AND carM, FOR USE IN STREPTOMYCES AND OTHER ORGANISMS

The present invention comprises novel carbomycin biosynthetic genes, designated carL and carM, methods for using the carL and carM genes, recombinant DNA cloning vectors that comprise the novel genes, and transformants containing the novel vectors. Streptomyces thermotolerans (ATCC 11416) produces carbomycin, a macrolide antibiotic consisting of a 16-member cyclic lactone and two sugar residues. The antibiotic activity of carbomycin, like that of other macrolides, is due to inhibition of protein synthesis by a mechanism that involves the binding of carbomycin to the ribosome. The carL and carM genes encode sugar biosynthesis or addition activities required to produce functional antibiotic.

The present invention provides carbomycin biosynthetic gene expression vectors for use in Streptomyces and other host cells. The development and exploitation of recombinant DNA technology in Streptomyces has been driven by the desire to improve the antibiotic-producing ability of this industrially important organism, not only to increase antibiotic yield, but also to produce novel antibiotics. This development has been somewhat retarded by the low number of antibiotic biosynthetic genes presently available for use in modifying Streptomyces by recombinant DNA technology. The present invention is useful and especially important in that it expands the number of antibiotic biosynthetic genes suitable for such use.

The vectors of the present invention are particularly useful, because the vectors can be introduced into and selected for in a variety of Streptomyces cells. Streptomyces provided over half of the clinically important antibiotics and thus is a commercially significant group. The present invention provides new and useful vectors and methods not only for this industrially important group but also for other antibiotic-producing organisms and allows for increasing the yield of antibiotic in fermentations and also for producing new antibiotics and antibiotic derivatives.

For purposes of the present invention, as disclosed and claimed herein, the following terms are defined below.

Am$^R$ - the apramycin resistance-conferring gene.

Antibiotic - a substance produced by a microorganism which, either naturally or with limited modification, will inhibit the growth of or kill another microorganism or eukaryotic cell.

Antibiotic Biosynthetic Gene - a DNA segment that encodes one or more activities that are necessary in the biochemical process of converting primary metabolites into antibiotics.

Antibiotic Biosynthetic Pathway - the entire set of antibiotic biosynthetic genes necessary for the process of converting primary metabolites into antibiotics.

Antibiotic-Producing Organism - any organism, including, but not limited to, Actinoplanes, Actinomadura, Bacillus, Cephalosporium, Micromonospora, Penicillium, Nocardia, and Streptomyces, which either produces an antibiotic or contains genes which, if expressed, would produce an antibiotic.

Antibiotic Resistance-Conferring Gene - a DNA segment that encodes an enzymatic or other activity that confers resistance to an antibiotic.

Ap$^R$ - the ampicillin resistance-conferring gene.

Bifunctional Cloning Shuttle Vector - a recombinant DNA cloning vector that can replicate and/or integrate into organisms of two different taxa.

Cloning - the process of incorporating a segment of DNA into a recombinant DNA cloning vector and transforming a host cell with the recombinant DNA.

carA - a carbomycin resistance-conferring gene of type A.

carB - a carbomycin resistance-conferring gene of type B. carG - a DNA sequence that comprises one or more genes that encode the activities required to form the 16-member cyclic lactone of carbomycin.

carL - a DNA sequence that comprises one or more genes that encode the activities required to allow Streptomyces fradiae GS33 to produce antibiotic.

carM - a DNA sequence that comprises one or more genes that encode the activities required to allow Streptomyces fradiae GS62 to produce antibiotic.

Constructed DNA - any DNA molecule which has been subjected to digestion by a restriction enzyme.

cos - the lambda cohesive end sequence.

Cosmid - a recombinant DNA cloning vector which not only can replicate in a host cell in the same manner as a plasmid but also can be packaged into phage heads.

Gene - A DNA sequence that comprises a promoter and coding sequence positioned so that the promoter drives transcription of the coding sequence.

Genetic Library - a set of recombinant DNA cloning vectors into which segments of DNA, comprising substantially all of the DNA of a particular organism, have been cloned.

Hybridization - the process of annealing two single-stranded DNA molecules to form a double-stranded DNA molecule, which may or may not be completely base-paired.

$Nm^R$ - the neomycin resistance-conferring gene.

ori - a plasmid origin of replication.

Phasmid--a recombinant DNA vector that may act as a phage or as a plasmid.

Recombinant DNA Cloning Vector - any autonomously replicating or integrating agent, including, but not limited to, plasmids, comprising a DNA molecule to which one or more additional DNA molecules can be or have been added.

Restriction Fragment - any linear DNA molecule generated by the action of one or more restriction enzymes.

rRNA - ribosomal ribonucleic acid.

Sensitive Host Cell - a host cell that cannot grow in the presence of a given antibiotic without a DNA segment that confers resistance thereto.

Sugar Biosynthesis Enzyme - any enzyme which mediates or directs the biosynthesis of a sugar, or any enzyme which mediates or directs the addition of a sugar to a pre-existing antibiotic or antibiotic-precursor molecule.

Sugar Biosynthesis Gene - any gene or genes which encode or regulate the expression of a sugar biosynthesis enzyme.

$Tc^R$ - the tetracycline-resistant phenotype or gene conferring same.

Transductant - a recipient host cell that has undergone transformation by recombinant phage infection.

Transformant - a recipient host cell that has undergone transformation.

Transformation - the introduction of DNA into a recipient host cell that changes the genotype and results in a change in the recipient cell.

$tsr^R$ - the thiostrepton-resistant phenotype or gene conferring same.

Figure 1 - the restriction site and function map of plasmid pOJ171. For the purposes of this disclosure, the Figures are not drawn exactly to scale.

Figure 2 - the restriction site and function map of plasmid pOJ160.

Figure 3 - the restriction site and function map of plasmid pOJ315.

Figure 4 - the restriction site and function map of plasmid pOJ220.

Figure 5 - the restriction site and function map of plasmid pOJ174.

Figure 6 - the restriction site and function map of plasmid pOJ182.

Figure 7 - the restriction site and function map of plasmid pOJ226.

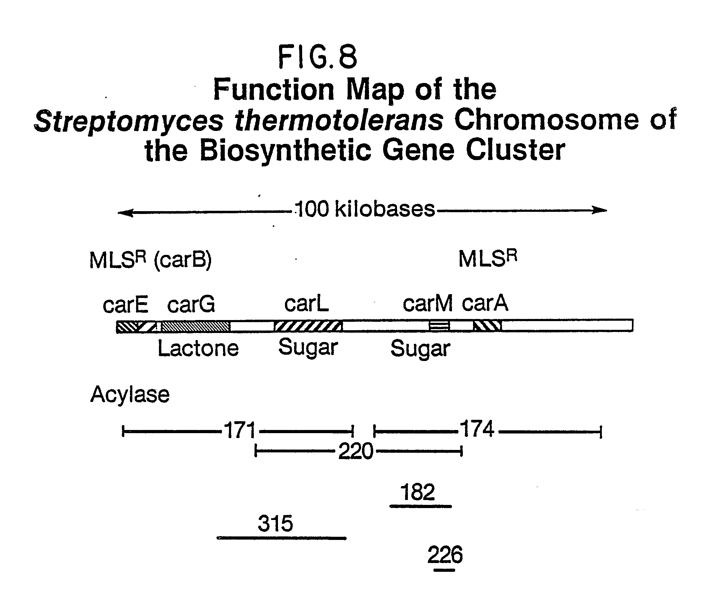

Figure 8 - the function map of the Streptomyces thermotolerans chromosome of the carbomycin biosynthetic gene cluster.

The present invention comprises a novel carbomycin biosynthesis gene, designed carL, as well as a method for increasing the amount of a sugar biosynthesis enzyme in an organism, said method comprising:

a) transforming said organism with a recombinant DNA vector that codes for expression of the carL gene product, and

b) culturing said organism transformed in step (a) under conditions that allow for gene expression.

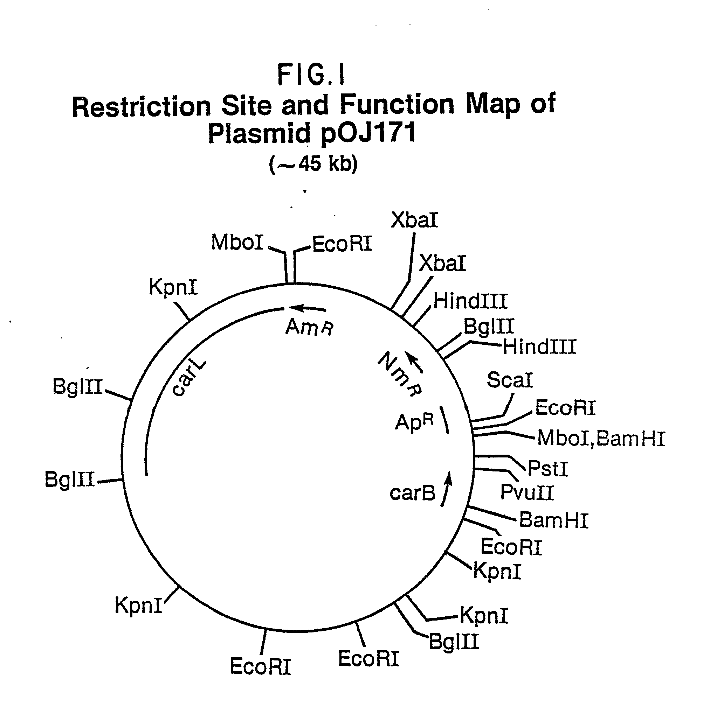

The carL gene can be isolated from plasmid pOJ171 on an ~20.0 kb EcoRI restriction fragment. Plasmid pOJ171 can be isolated from E. coli K12 SF8/pOJ171, a strain deposited on February 6, 1987 and made part of the permanent culture collection of the Agricultural Research Service, Northern Regional Research Center (NRRL), Peoria, IL 61604, under the accession number NRRL B-18169. A restriction site and function map of plasmid pOJ171 is presented in Figure 1 of the accompanying drawings. Plasmid pOJ171 can be isolated from E. coli K12 SF8/pOJ171 in substantial accordance with the procedure described in Example 1.

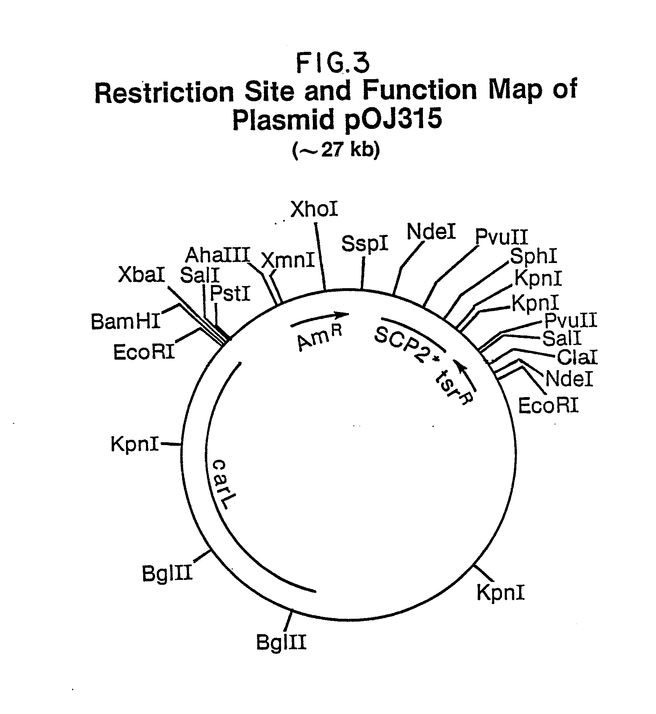

Plasmid pOJ171 serves as useful starting material for the construction of other vectors that contain the carL carbomycin biosynthetic gene. For example, the ~20.0 kb EcoRI, carbomycin biosynthetic gene-containing restriction fragment of plasmid pOJ171 was isolated and inserted into EcoRI-digested plasmid pOJ160 (NRRL B-18088 deposited July 29, 1986) to yield plasmids pOJ315 and pOJ315A, which differ only with respect to the orientation of the ~20.0 kb EcoRI, carbomycin biosynthetic-containing restriction fragment. The construction protocol for plasmids pOJ315 and pOJ315A is given in Example 2. A restriction site and function map of plasmid pOJ315 is presented in Figure 3 of the accompanying drawings.

The approximately 20 kb EcoRI restriction fragment of the present invention comprises the carL gene of Streptomyces thermotolerans. The activity of the carL gene is best understood by defining the mutation which the carL gene complements. Certain mutants of Streptomyces fradiae, such as S. fradiae GS33 (NRRL 18420 deposited September 23, 1988) do not produce the antibiotic, tylosin. Such mutants lack a functional tylL gene and therefore cannot produce sugar biosynthesis enzymes needed to produce the final antibiotic. These mutants, however, can produce antibiotic upon transformation with a vector comprising the carL gene of the present invention. The carL gene, therefore, is a sugar biosynthesis gene which enables the host to produce the sugar biosynthesis enzymatic activities which allow the S. fradiae GS33 mutant to complete the biosynthesis of antibiotic. Similarly, when the carL gene of the present invention is introduced into other strains of macrolide-producing Streptomyces, these transformed organisms produce a variety of hybrid antibiotics.

The present invention further comprises a novel carbomycin biosynthesis gene, designed carM, as well as a method for increasing the amount of sugar biosynthesis enzyme in an organism, said method comprising:

a) transforming said organism with a recombinant DNA vector that codes for expression of the carM gene product, and

b) culturing said organism transformed in step (a) under conditions that allow for gene expression.

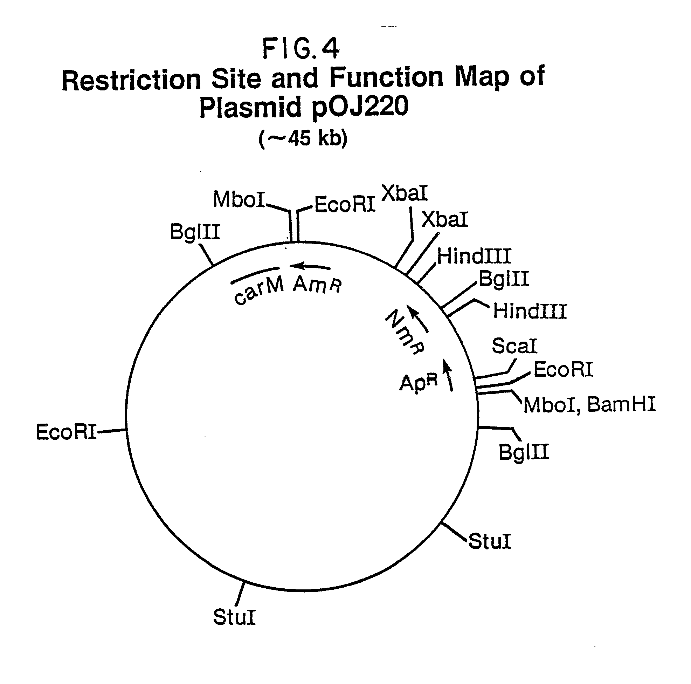

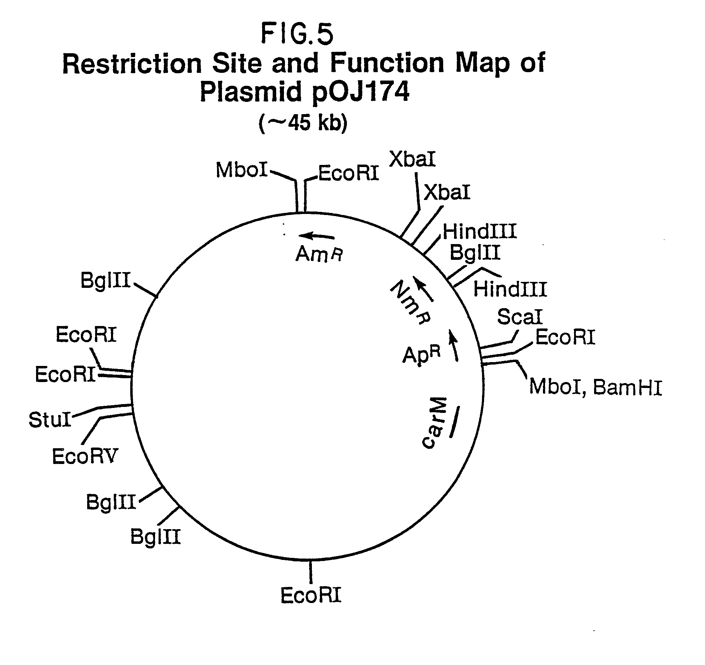

The carM gene can be isolated from plasmid pOJ174 on an ~10Kb EcoRI restriction fragment. Plasmid pOJ174 can be isolated from E. coli K12 SF8/pOJ174, a strain deposited on June 4, 1987 and made part of the permanent culture collection of the NRRL under the accession number NRRL B-18221. A restriction site and function map of plasmid pOJ174 is presented in Figure 5 of the accompanying drawings. The carM gene can also be isolated on the same ~10Kb EcoRI restriction fragment and on plasmid pOJ220, whi.ch can be isolated from E. coli K12 SF8/pOJ220, a strain deposited on June 4, 1987 and available from the NRRL under the accession number NRRL B-18222. A restriction site and function map of plasmid pOJ220 is presented in Figure 4 of the accompanying drawings.

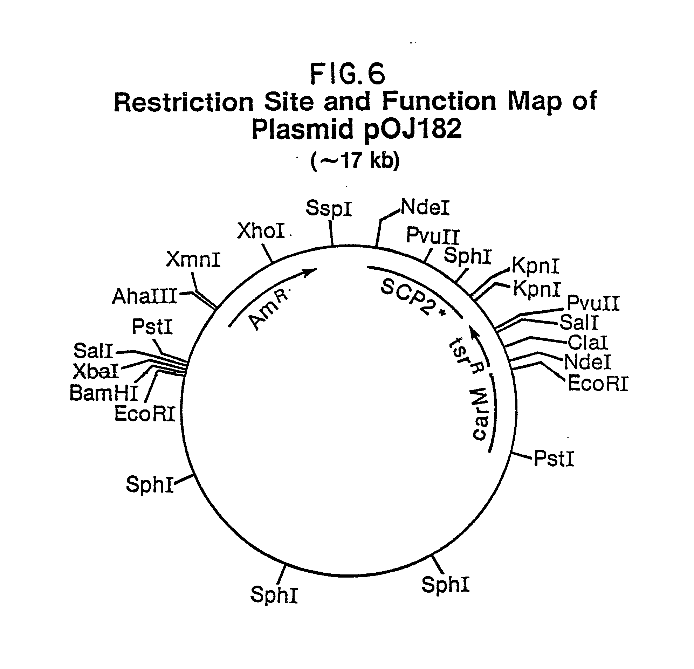

Plasmid pOJ174 serves as useful starting material for the construction of other vectors that contain the carM carbomycin biosynthetic gene. For example, the ~10Kb EcoRI, carbomycin biosynthetic gene-containing restriction fragment of plasmid pOJ174 was isolated and inserted into EcoRI-digested plasmid pOJ160 to yield plasmids pOJ182 and pOJ182A, which differ only with respect to the orientation of the ~10Kb EcoRI, carbomycin biosynthetic-containing restriction fragment The construction of plasmids pOJ182 and pOJ182A is described in greater detail in Example 5. A restriction site and function map of plasmid pOJ182 is presented in Figure 6 of the accompanying drawings.

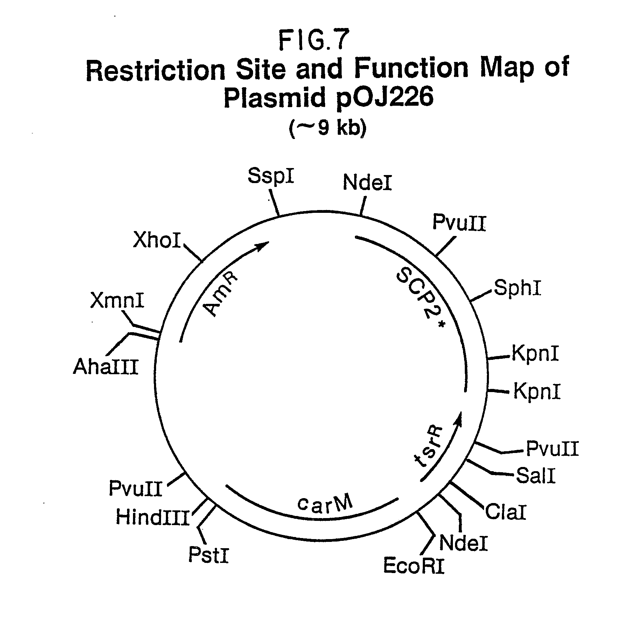

Plasmid pOJ182 may itself be used to construct still other useful vectors which comprise the carM carbomycin biosynthetic gene. A PstI digestion and deletion of plasmid pOJ182 yielded plasmid pOJ226, which contains the carM carbomycin biosynthetic gene on an ~2.0 kb EcoRI-PstI restriction fragment. The construction protocol for plasmid pOJ226 is described in greater detail in Example 6. A restriction site and function map of plasmid pOJ226 is presented in Figure 7 of the accompanying drawings.

The approximately 2.0 kb EcoRI-PstI restriction fragment of the present invention comprises the carM gene of Streptomyces thermotolerans. The carM carbomycin biosynthetic gene of the present invention complements the Streptomyces fradiae mutant, S. fradiae GS62 (NRRL 18421 deposited September 23, 1988), which cannot produce the macrolide antibiotic, tylosin. Streptomyces fradiae GS62 is a member of a class of mutants which lack a functional tylM gene and therefore cannot produce sugar biosynthesis enzymes needed to produce the final antibiotic. Such mutants, however, can produce antibiotic upon transformation with a vector comprising the carM gene. The carM gene, therefore, is a sugar biosynthesis gene which enables the host to produce the sugar biosynthesis enzymatic activities which allow the S. fradiae GS62 mutant to complete the biosynthesis of antibiotic. Furthermore, when the carM gene of the present invention is introduced into other strains of macrolide-producing Streptomyces, these transformed organisms produce a variety of hybrid antibiotics.

The carL and carM genes were isolated from a carbomycin-producing strain of Streptomyces thermotolerans (ATCC 11416). Thus, genomic DNA of S. thermotolerans was partially digested with restriction enzyme MboI, and the resulting DNA was inserted into HpaI-BamHI-digested cosmid pKC462A to yield a number of carL and carM-containing plasmids, including plasmids pOJ171, pOJ220 and pOJ174. Plasmid pKC462A (NRRL B-15973) was deposited at the NRRL on June 3, 1985.

The vectors of the present invention can be used to transform a variety of organisms to increase the antibiotic-producing ability of the organism or to allow the organisms to complete the biosynthesis of antibiotics or antibiotic precursors. In addition, the carL and carM genes can be used to transform a variety of antibiotic-producing organisms, particularly macrolide antibiotic-producing organisms, for purposes of making novel antibiotics. The carL and carM genes can be reconstructed using recombinant DNA techniques for purposes of producing the carL and carM gene products in non-Streptomyces species. The following Tables present a representative sampling of various antibiotic-producing organisms in which the carL and carM genes can be used either to produce a novel antibiotic or to increase antibiotic production.

TABLE I

| Ansamycin Antibiotic-Producing Organisms | |
|---|---|
| Organism | Antibiotic |
| Micromonospora | |
| various species | various ansamycins |
| Nocardia | |
| mediterranei | rifamycin |
| Streptomyces | |
| collinus<br>diastochromogenes<br>galbus subsp. griseosporeus<br>hygroscopicus<br>hygroscopicus var. geldanus var. nova<br>nigellus<br>rishiriensis<br>sp. E/784<br>sp. E88<br>spectabilis<br>tolypophorous | ansatrienes and napthomycins<br>ansatrienes and napthomycins<br>napthomycin B<br>herbimycin<br>geldamycin<br>21-hydroxy-25-demethyl 25-methylthioprotostreptovaricin<br>mycotrienes<br>actamycin and mycotrienes<br>mycotrienes<br>streptovaricins<br>tolypomycin |

TABLE II

| Anthracycline and Quinone Antibiotic-Producing Organisms | |
|---|---|
| Organism | Antibiotic |
| Streptomyces | |
| caespitosus<br>coelicolor<br>coeruleorubidicus<br>cyaneus<br>flavogriseus<br>galilaeus<br>lusitanus<br>peuceticus<br>violochromogenes | mitomycins A, B, and C<br>actinorhodin<br>daunomycin<br>ditrisarubicin<br>cyanocycline A<br>aclacinomycin A, auramycins, and sulfurmycins<br>napthyridinomycin<br>daunomycin and adriamycin<br>arugomycin |

5

## TABLE III

Macrolide, Lincosamide, and Streptogramin
Antibiotic-Producing Organisms

| Organism | Antibiotic |
|---|---|
| <u>Micromonospora</u> | |
| <u>rosaria</u> | rosaramicin |
| | |
| <u>Streptomyces</u> | |
| <u>albireticuli</u> | carbomycin |
| <u>albogriseolus</u> | mikonomycin |
| <u>albus</u> | albomycetin |
| <u>albus</u> var. | |
| <u>coilmyceticus</u> | coleimycin |
| <u>ambofaciens</u> | spiramycin and foromacidin D |
| <u>antibioticus</u> | oleandomycin |
| <u>avermitilis</u> | avermectins |
| <u>bikiniensis</u> | chalcomycin |
| <u>bruneogriseus</u> | albocycline |
| <u>caelestis</u> | M188 and celesticetin |
| <u>cinerochromogenes</u> | cineromycin B |
| <u>cirratus</u> | cirramycin |
| <u>deltae</u> | deltamycins |
| <u>djakartensis</u> | niddamycin |
| <u>erythreus</u> | erythromycins |
| <u>eurocidicus</u> | methymycin |
| <u>eurythermus</u> | angolamycin |
| <u>fasciculus</u> | amaromycin |
| <u>felleus</u> | argomycin and picromycin |
| <u>fimbriatus</u> | amaromycin |
| <u>flavochromogenes</u> | amaromycin and shincomycins |
| <u>fradiae</u> | tylosin |
| <u>fungicidicus</u> | NA-181 |
| <u>fungicidicus</u> var. | |
| <u>espinomyceticus</u> | espinomycins |
| <u>furdicidicus</u> | mydecamycin |
| <u>goshikiensis</u> | bandamycin |
| <u>griseofaciens</u> | PA133A and B |
| <u>griseoflavus</u> | acumycin |
| <u>griseofuscus</u> | bundlin |
| <u>griseolus</u> | griseomycin |
| <u>griseospiralis</u> | relomycin |
| <u>griseus</u> | borrelidin |

6

## TABLE III (Continued)

### Macrolide, Lincosamide, and Streptogramin Antibiotic-Producing Organisms

| Organism | Antibiotic |
|---|---|
| Streptomyces | |
| griseus ssp. sulphurus | bafilomycins |
| halstedi | carbomycin and leucanicidin |
| hygroscopicus | tylosin |
| hygroscopicus subsp. aureolacrimosus | milbemycins |
| kitastoensis | leucomycin $A_3$ and josamycin |
| lavendulae | aldgamycin |
| lincolnensis | lincomycin |
| loidensis | vernamycin A and B |
| macrosporeus | carbomycin |
| maizeus | ingramycin |
| mycarofaciens | acetyl-leukomycin, and espinomycin |
| narbonensis | josamycin and narbomycin |
| narbonensis var. josamyceticus | leucomycin $A_3$ and josamycin |
| olivochromogenes | oleandomycin |
| platensis | platenomycin |
| rimosus | tylosin and neutramycin |
| rochei | lankacidin and borrelidin |
| rochei var. volubilis | T2636 |
| roseochromogenes | albocycline |
| roseocitreus | albocycline |
| spinichromogenes var. suragaoensis | kujimycins |
| tendae | carbomycin |
| thermotolerans | carbomycin |
| venezuelae | methymycins |
| violaceoniger | lankacidins and lankamycin |

TABLE IV

| Miscellaneous Antibiotic-Producing Streptomyces | | |
|---|---|---|
| Antibiotic Type | Streptomyces Species | Antibiotic |
| cyclopentane ring-containing | coelicolor | methylenomycin A |
| | erythrochromogenes | sarkomycin |
| | kasugaensis | aureothricin and thiolutin |
| | violaceoruber | methylenomycin A |
| polyenes | griseus | candicidin |
| | nodosus | amphotericin B |
| | noursei | nystatin |
| tetracyclines | aureofaciens | tetracycline, chlortetracycline, demethyltetracycline, and demethylchlortetracycline |
| | rimosus | oxytetracycline |

TABLE V

| Polyether Antibiotic-Producing Organism | |
| --- | --- |
| Organism | Antibiotic |
| Actinomadura | |
| various species oligosporus | various polyethers A80190 |
| Dactylosporangium | |
| various species | various polyethers |
| Nocardia | |
| various species | various polyethers |
| Streptomyces | |
| albus aureofaciens bobili cacaoi var. asoensis chartreusis cinnamonensis conglobatus eurocidicus var. asterocidicus flaveolus gallinarius griseus hygroscopicus lasaliensis longwoodensis mutabilis pactum ribosidificus violaceoniger | A204, A28695A and B, and salinomycin narasin A80438 lysocellin A23187 monensin ionomycin laidlomycin CP38936 RP 30504 grisorixin A218, emericid, DE3936, A120A, A28695A and B, etheromycin, and dianemycin lasalocid lysocellin S-11743a A80438 lonomycin nigericin |
| Streptoverticillium | |
| various species | various polyethers |

The carL and carM genes function particularly well in Streptomyces fradiae GS33 and S. fradiae GS62, respectively. Yet even if the original carL and carM genes fail to express in a given organism, such as E. coli, because, for example, the Streptomyces promoter failed to function in that organism, the carL and carM coding sequences of the present invention could be ligated to a DNA containing an appropriate promoter and ribosome-binding site to achieve expression of the carL or carM genes in the host of choice.

Plasmid pOJ315 contains the carL gene: (1) a promoter that directs transcription of the protein-coding sequence; (2) a sequence that, when transcribed into mRNA, directs translation of the transcript; (3) a protein-coding sequence; and (4) a transcription terminator. Plasmid pOJ182 contains each of these four elements of the carM gene. Each of these elements is independently useful and can, through the techniques of recombinant DNA technology, be used to form recombinant genes of great variety. The DNA sequence of the carL and carM genes will reveal the location of the carL and carM coding sequences and thus allow one to position other promoters and translational control sequences, for example, the trp, lpp, and lac promoters and translational control sequences of E. coli, the hybrid tac promoter and translational control sequence, and the veg promoter and translational control sequence of Bacillus, in reading phase

EP 0 361 905 A2

with the carL or carM coding sequence. By choosing the proper promoter and translational control sequence, one can construct vectors that drive expression of the carL or carM gene products in any host cell. The promoters of the carL and carM genes are useful in their own right. The promoters and other regulatory elements of the carL and carM genes can be linked to the coding sequences of non-carbomycin antibiotic biosynthetic genes to prepare hybrid antibiotic pathway genes that function in other Streptomyces species to yield hybrid antibiotics. Thus, the individual elements of the gene on the plasmids described herein comprise important components of the present invention.

Those skilled in the art will recognize that the carL sequence deposited under accession number NRRL B-18169 and the carM sequence deposited under accession number NRRL B-18221 can be used to prepare DNA probes for use in obtaining other biosynthetic gene-containing DNA segments, especially segments encoding macrolide biosynthetic genes. In addition, due to the diversity of Streptomyces thermotolerans strains both in nature and also in the laboratory, there may be a variety of allelic variants of the carL and carM genes that can be readily isolated given the carL and carM gene-containing compounds of this invention. These allelic variants, which encode gene products that are functionally equivalent to the carL or carM gene disclosed herein also fall within the scope of the present invention.

A variety of known Streptomyces replicons can be used in conjunction with the carL and carM genes to construct expression vectors of the present invention. Table VI is an illustrative, but not comprehensive, listing of Streptomyces plasmids from which Streptomyces replicons can be obtained. Those skilled in the art recognize that, so long as the replicon function is not disrupted, all or part of the plasmids can be used to construct vectors that contain the carL and carM genes of the present invention. The plasmid-containing host and depository accession number are also listed in Table VI.

Table VI

| Streptomyces Plasmids | | |
|---|---|---|
| Plasmid | Host | Accession Number |
| SCP2 | Streptomyces coelicolor A3(2) | NRRL 15042 |
| SCP2" | Streptomyces coelicolor M110 | NRRL 15041 |
| pEL7 | Streptomyces ambofaciens/pEL7 | NRRL 12523 |
| pUC6 | Streptomyces espinosus | NRRL 11439 |
| pUC3 | Streptomyces 3022A | NRRL 11441 |
| SLP1 | Streptomyces lividans | NCIB* 11417 |
| pNM100 | Streptomyces virginiae | NRRL 15156 |
| pEL103 | Streptomyces granuloruber A399 12.13/pEL103 | NRRL 12549 |
| pIJ702 | Streptomyces lividans | ATCC** 39155 |

*National Collection of Industrial Bacteria (NCIB), Torry Research Station, Post Office Box 31, 135 Abbey Road, Aberdeen AB98DG, Scotland, United Kingdom.
**American Type Culture Collection, Rockville, MD 20852.

Restriction fragments used to construct vectors illustrative of the present invention can be conventionally modified to facilitate ligation. For example, molecular linkers can be provided to a particular carL or carM biosynthetic gene-containing restriction fragment or to DNA comprising vector replication or integration functions. Thus, specific sites for subsequent ligation can be conveniently constructed. In addition, the various carL or carM biosynthetic gene-containing restriction fragments, origin of replication, or sequences that provide for chromosomal integration of a given vector can be modified by adding, eliminating, or substituting certain nucleotides to alter characteristics and to provide a variety of restriction sites for ligation of DNA. Those skilled in the art understand nucleotide chemistry and the genetic code and thus which nucleotides are interchangeable and which DNA modifications are desirable for a specific purpose. It is also noteworthy that a given carL or carM biosynthetic gene-containing restriction fragment is not limited to a particular position on a cloning vector, as long as critical, vector-controlled functions are not disrupted. Those skilled in the art understand or can readily determine which sites on a vector are advantageous for the ligation or insertion of a particular carL or carM gene-containing restriction fragment.

Of course, the carL and carM genes can be used to construct vectors other than plasmids. Phage ØC31 is a well-known Streptomyces phage that is an excellent source of starting material for constructing integrative carbomycin biosynthetic gene-containing vectors that further exemplify the present invention. A derivative of phage ØC31, phasmid pKC331, is especially preferred for constructing such integrating vectors and can be obtained from E. coli K12 BE447/pKC331 deposited August 3, 1984 (NRRL B-15828). ØC31-type phages are integrative vectors and can be readily modified to incorporate the carL or carM genes and thus confer carL or carM activity to Streptomyces. Even plasmids that contain a replicon that provides for extrachromosomal maintenance of the plasmid sometimes integrate into the genome of the host cell, usually with concomitant deletion of the replicon sequences. The present invention thus is not limited by the type of vector used to introduce the carL and carM genes into the target host cell nor by the location of the carL or carM genes once introduction has occurred.

The vectors of the present invention comprise a Streptomyces replicon and a carL or carM gene-containing restriction fragment. Because amplification and manipulation of plasmids is done faster and more efficiently in E. coli than in Streptomyces, it is convenient to add DNA sequences that also allow for replication in E. coli. Thus, the addition of functional replicon-containing and antibiotic resistance-conferring restriction fragments from E. coli plasmids such as, for example, pUC8, pUC18, pUC19, pBR322, pACYC184, pBR325, pBR328, and the like is highly advantageous and adds to the general utility of the present illustrative vectors.

Streptomyces thermotolerans contains two carbomycin resistance-conferring genes, designated carA and carB. These two carbomycin resistance genes may act in concert to cause high-level resistance in Streptomyces thermotolerans. The carA gene is disclosed and claimed in, EPP et al., U.S. Patent Application Serial No. 901,240, attorney docket No. X-6935, filed August 28, 1986, while the carB gene is disclosed and claimed in EPP et al., U.S. Patent Applicaiton Serial Number 901,334, attorney docket No. X-7135, filed August 28, 1986 (see European Patent Appl. 87307538.6, Pub. Number 0258046). The present invention also comprises vectors that contain the carL or carM genes and either or both of the carA and carB genes. Plasmid pOJ171, for example, comprises both the carL and carB genes, while plasmid pOJ174 comprises both the carM and carA genes.

The cloning vectors and transformants of the present invention provide for the cloning of genes to improve yields of various products that are currently produced in Streptomyces and related cells. Examples of such products include, but are not limited to, Carbomycin Streptomycin, Tylosin, Cephalosporins, Actaplanin, Narasin, Monensin, Tobramycin, Erythromycin, and the like. The present invention also provides selectable vectors that are useful for cloning, characterizing, and reconstructing a variety of useful DNA sequences.

Streptomyces can be cultured in a number of ways using any of several different media. Preferred carbohydrate sources in a culture medium include, for example, molasses, glucose, dextrin, and glycerol. Nitrogen sources include, for example, soy flour, amino acid mixtures, and peptones. Nutrient inorganic salts are also incorporated and include the customary salts capable of yielding sodium, potassium, ammonium, calcium, phosphate, chloride, sulfate, and like ions. As is necessary for the growth and development of other microorganisms, essential trace elements are also added. Such trace elements are commonly supplied as impurities incidental to the addition of other constituents of the medium.

Streptomyces is grown under aerobic culture conditions over a relatively wide pH range of about 5 to 9 at temperatures ranging from about $15°$ to $40°$ C. For plasmid stability and maintenance, it is desirable to start with a culture medium at a pH of about 7.2 and maintain a culture temperature of about $30°$ C.

The following examples further illustrate and describe the invention disclosed herein. The invention is not limited in scope by reason of any of the following Examples; sources of reagents or equipment are provided merely for convenience and in no way limit the invention. Both an explanation of and the actual procedures for constructing the invention are described where appropriate.

Example 1

Isolation of Plasmid pOJ171

A. Culture of E. coli K12 SF8/pOJ171

Plasmid pOJ171 can be obtained from the Northern Regional Research Center in E. coli K12 SF8 under the accession number NRRL B-18169 which was deposited on February 6, 1987. The lyophils of E. coli K12 SF8/pOJ171 are plated onto L-agar plates (10 g of Bacto-tryptone, 10 g of NaCl, 5 g of Bacto-Yeast Extract, and 15 g of agar per liter) containing 200 μg/ml apramycin to obtain a single colony isolate of the strain. This colony is used to inoculate about 500 ml of L broth (L agar without agar) containing 200 μg/ml apramycin, and the resulting culture is incubated at 37°C with aeration until the cells reach stationary phase.

Plasmid DNA was obtained from the cells to use in construction of plasmid pOJ325 in accordance with the following procedure, which is adapted from Maniatis et al., 1982, Molecular Cloning (Cold Spring Harbor Laboratory). This same procedure was used, but on a smaller scale and with the ultracentrifugation steps replaced with phenol followed by chloroform extractions, to prepare the plasmid DNA used to identify the E. coli K12 RR1ΔM15/pOJ325 transformants.

About 500 ml of stationary-phase E. coli/pOJ171 cells are harvested by centrifugation at 4000Xg for 10 minutes at 4°C, and the supernatant is discarded. The cell pellet is washed in 100 ml of ice-cold STE buffer (0.1 M NaCl; 10 mM Tris-HCl, pH 7.8; and 1 mM EDTA). After the cell pellet is washed, the pellet is resuspended in 10 ml of Solution 1 (50 mM glucose; 25 mM Tris-HCl, pH = 8.0; and 10 mM EDTA) that contains 1 mg/ml lysozyme and is left at room temperature for 10 minutes. Twenty ml of Solution 2 (0.2 N NaOH and 1% SDS) are then added to the lysozyme-treated cells, and the solution is gently mixed by inversion. The mixture is incubated on ice for 10 minutes.

Fifteen ml of ice-cold, 3 M sodium acetate, pH = 4.8, are added to the lysed-cell mixture, and the solution is mixed by inversion. The solution is incubated on ice for 60 minutes. The. 3 M sodium acetate solution is prepared by mixing equal volumes of 3 M acetic acid and 3 M sodium acetate.

The lysed cell mixture is centrifuged in a Beckman SW27 rotor (or its equivalent) at 20,000 rpm for 20 minutes at 4°C. About 36 ml of supernatant are recovered, and 2.5 volumes of ethanol are added, mixed, and the resulting solution left on ice for 15 minutes. The plasmid DNA is collected by centrifugation at 12,000Xg for 30 minutes at room temperature. The supernatant is discarded, and the DNA pellet is washed with 70% ethanol at room temperature. The ethanol wash is decanted, and the pellet is dried in a vacuum desiccator. The pellet is then resuspended in 8 ml of TE buffer (10 mM Tris-HCl, pH = 8.0, and 1 mM EDTA).

Eight grams of CsCl are added to the DNA solution. About 0.8 ml of a 10 mg/ml solution of ethidium bromide in water are added for each 10 ml of CsCl-DNA solution. The final density of the solution is about 0.761 g/ml, and the ethidium bromide concentraton is about 800 μg/ml. The solution is transferred to a Beckman Type 50 centrifuge tube, filled to the top with TE buffer containing 0.761 g of CsCl per ml, sealed, and centrifuged at 45,000 rpm for 24 hours at 20°C. After centrifugation, two bands of DNA are visible in ordinary light and become even more prominent in UV light. The cap is removed from the tube, and the lower DNA band is recovered using a syringe with a #21 hypodermic needle inserted through the side of the centrifuge tube.

The ethidium bromide is removed from the solution of plasmid DNA by several extractions with water-saturated 1-butanol, and the CsCl is removed by dialysis against TE buffer. After extractions with buffered phenol and then chloroform, the DNA is precipitated, washed with 70% ethanol, and dried. About 0.5 mg of plasmid pOJ171 DNA can be obtained by this procedure. A restriction site and function map of plasmid pOJ171 is presented in Figure 1 of the accompanying drawings.


Example 2


Construction of Plasmid pOJ315 and pOJ315A


A. Isolation of Plasmid pOJ160

Plasmid pOJ160 can be obtained from the Northern Regional Research Center in E. coli K12 JM109 under the accession number NRRL B-18088. The lyophils of E. coli K12 JM109/pOJ160, which was deposited on July 29, 1986, are plated onto L-agar plates (10 g of Bacto-tryptone, 10 g of NaCl, 5 g of Bacto-Yeast Extract, and 15 g of agar per liter) containing 200 μg/ml apramycin to obtain a single colony isolate of the strain. This colony is used to inoculate about 500 ml of L broth (L agar without agar) containing 200 μg/ml apramycin, and the resulting culture is incubated at 37°C with aeration until the cells reach stationary phase.

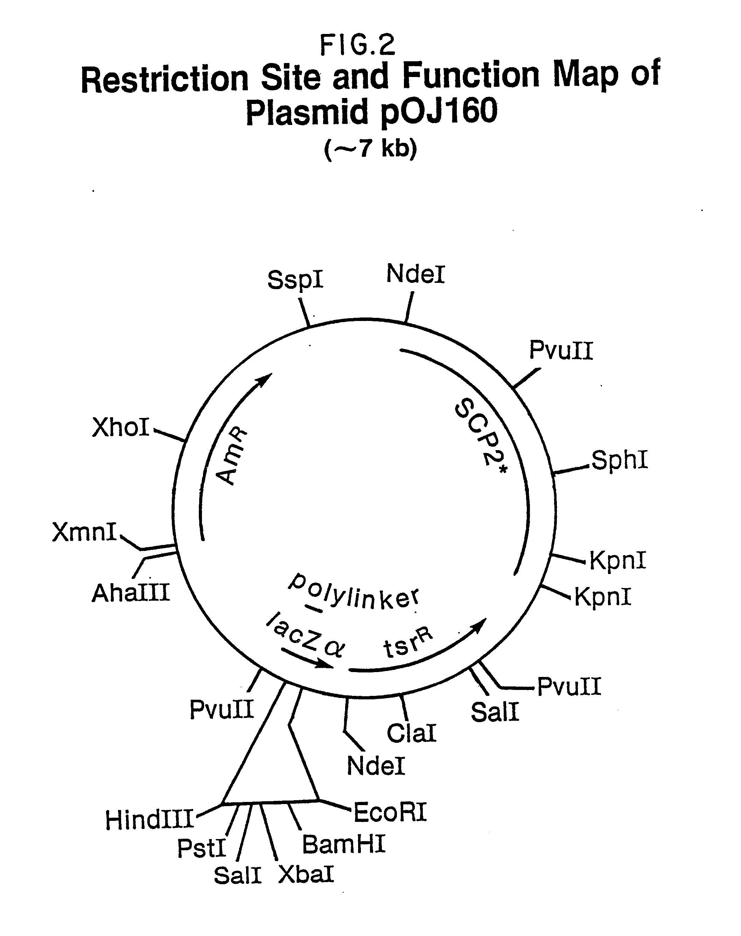

Plasmid DNA was obtained from the cells to use in construction of plasmid pOJ315 in accordance with the procedure set forth in Example 1, above. About 0.5 mg of plasmid pOJ160 DNA can be obtained by this procedure. A restriction site and function map of plasmid pOJ160 is presented in Figure 2 of the accompanying drawings.

### B. Final Construction of Plasmid pOJ315

About 10 μg (10 μl) of plasmid pOJ160 DNA were added to 2 μl of 10X EcoRI buffer (1 M Tris-HCl, pH = 7.5; 500 mM NaCl; 1 mg/ml BSA and 50 mM MgCl₂), 6 μl of H₂O, and 2 μl (~30 units; unit definitions herein correspond to those of New England Biolabs, 32 Tozer Road, Beverly, MA 01915-9990, unless otherwise indicated) of restriction enzyme EcoRI. The resulting reaction was incubated at 37°C for two hours. The EcoRI-digested plasmid pOJ160 DNA was collected by adjusting the sodium acetate (NaOAc) concentration of the reaction mixture to 0.30 M, adding 2.5 volumes of ethanol, chilling the reaction mixture to -70°C, and centrifuging to pellet the precipitated DNA. The pellet of EcoRI-digested plasmid pOJ160 DNA was resuspended in 400 μl of TE buffer (10 mM Tris-HCl, pH = 8.0, and 1 mM EDTA). About 1 μl (0.1 unit) of bacterial alkaline phosphatase (International Biotechnology, Inc., P.O. Box 1565, New Haven, CT 06506) was added to the DNA solution, and the reaction was incubated at 65°C for 1 hour. The reaction mixture was extracted with 400 μl of a 1:1 solution of phenol:chloroform and then extracted with 400 μl of chloroform. The EcoRI-digested, dephosphorylated plasmid pOJ160 DNA was collected by ethanol precipitation and centrifugation as described above, and the DNA pellet was resuspended in 10 μl of TE buffer.

About 10 μg of plasmid pOJ171 in 100 μl of TE buffer were added to 13 μl of 10X EcoRI buffer 13 μl of H₂O and 4 μl (~60 units) of restriction enzyme EcoRI. The resulting reaction was incubated at 37°C for 2 hours. The reaction mixture was extracted, and the DNA was collected as described above. The pelleted DNA was then resuspended and digested with restriction enzyme AhaIII in substantial accordance with the above teaching, except 10X AhaIII buffer (100 mM Tris-HCl (pH 8.0), 1 M NaCl and 100 mM MgCl₂) and restriction enzyme AhaIII was used. Following a one hour incubation at 37°C, the DNA was again precipitated. The DNA pellet was dissolved in 50 μl of TE buffer and contained ~2.5 μg of the desired ~20 kb EcoRI restriction fragment of plasmid pOJ171. The EcoRI-digested, dephosphorylated plasmid pOJ160 DNA (1 μl) was added to 10 μl (0.5 μg) of the EcoRI-AhaIII-digested plasmid pOJ171 DNA, 2 μl of 10X ligase buffer (660 mM Tris-HCl, pH = 8; 66 mM MgCl₂; 10 mM dithiothreitol (DTT); and 10 mM ATP), and 6 μl of H₂O. About 1 μl (~100 units) of T4 DNA ligase was added to the solution of DNA, and the resulting reaction was incubated at 15°C overnight (~16 hours). The ligated DNA contained the desired plasmid pOJ315. A restriction site and function map of plasmid pOJ315 is presented in Figure 3 of the accompanying drawings. Because the ~20 kb, carL gene-containing EcoRI restriction fragment of plasmid pOJ171 could insert into plasmid pOJ160 in either of two orientations, the ligation also produced plasmid pOJ315A, which differs from plasmid pOJ315 only with respect to the orientation of the carL gene-containing restriction fragment.

The EcoRI site on plasmid pOJ160 resides within a polylinker that itself forms part of the DNA sequence encoding the lacZ α-fragment. Expression of the lacZ α-fragment in an E. coli ΔM15 strain, such as E. coli K12 RR1ΔM15 (NRRL B-15440 deposited May 5, 1983), restores the strain's ability to produce a functional β-galactosidase enzyme. Thus, plasmid pOJ160 can restore β-galactosidase activity to the E. coli K12 RR1ΔM15 strain. However, insertion of DNA into a restriction site of the polylinker on plasmid pOJ160, as occurs in the construction of plasmid pOJ315, disrupts the lacZ α-fragment coding sequence and concomitantly destroys the ability of the plasmid pOJ160 derivative to complement the ΔM15 mutation. β-galactosidase can hydrolyze X-Gal, which is 5-bromo-4-chloro-3-indolyl-β-D-galactopyranoside, a colorless compound, to an indigo-colored product and thus allows for a convenient screening method for discriminating between transformants containing starting plasmid pOJ160 and those containing a plasmid pOJ160 derivative, such as plasmid pOJ315.

To prepare E. coli K12 RR1ΔM15 cells that are competent for transformation, the lyophils of E. coli K12 RR1ΔM15 obtained from the NRRL are reconstituted to isolate single colonies. One single-colony isolate of RR1ΔM15 was inoculated into 10 ml of L broth (10 g of Bacto-tryptone, 10 g of NaCl, and 5 g of Bacto-Yeast Extract per liter), and the culture was incubated at 37°C overnight with aeration. The overnight culture was used to inoculate 200 ml of L broth to yield a culture with an O.D.₆₀₀ of about 0.1. The culture was incubated at 37°C with aeration until the O.D.₆₀₀ was about 0.6. The culture was collected by centrifugation at 4000Xg for 10 minutes at 4°C, resuspended in 100 ml of cold 50 mM CaCl₂, and incubated on ice for 15 to 30 minutes.

The cells were again collected by centrifugation and resuspended in 10 ml of cold 50 mM CaCl₂ containing 20% glycerol. A 200 $\mu$l aliquot of the cells was added to the ligated DNA prepared above. The cell-DNA mixture was incubated on ice for one hour, centrifuged, and the cell pellet was resuspended into 0.5 ml of L broth in a 1.5 ml tube and incubated with aeration at 37°C for one-half hour.

Aliquots of the transformation mixture were plated on L-agar (L-broth with 15 grams per liter agar) plates containing 200 $\mu$g apramycin/ml, 40 $\mu$g X-gal/ml, and 40 $\mu$g IPTG/ml. IPTG serves to derepress the expression of the $\alpha$-complementing fragment encoded on plasmid pOJ160. The plates were incubated at 37°C overnight. Colonies that contain a plasmid without an insert, such as E. coli K12 RR1$\Delta$M15/pOJ160, appear blue on these plates. Colonies that contain a plasmid with an insert, such as E. coli K12 RR1$\Delta$M15/pOJ315, are white. Several apramycin-resistant, white colonies were selected and then screened by restriction enzyme analysis of their plasmid DNA. Plasmid DNA was obtained from the E. coli K12 RR1$\Delta$M15/pOJ315 transformants in accordance with the procedure for isolating plasmid pOJ160 DNA, described above. The plasmid pOJ315 DNA can be used to transform Streptomyces fradiae, as described in Example 3, below.

## Example 3

Transformation of Streptomyces fradiae GS33 (NRRL 18420) to Produce S. fradiae GS33/pOJ315

### A. List of Solutions

The following solutions are referred to throughout the Examples and are presented here for clarity.

| 1. P Media (~100 ml): | |
|---|---|
| Ingredient | Amount |
| Sucrose | 10.3 g |
| $K_2SO_4$ | 0.025 g |
| Trace element solution (see #3) | 0.2 ml |
| $MgCl_2 \cdot 6H_2O$ | 0.203 g |
| Water | 80 ml |
| After autoclaving add: | |
| $KH_2PO_4$ (0.5%) | 1 ml |
| $CaCl_2 \cdot 2H_2O$ (3.68%) | 10 ml |
| (N-tris-(hydroxymethyl)methyl-2-aminoethane sulphonic acid), "TES" buffer, 0.25 M, pH = 7.2 | 10 ml |

| 2. Trace element solution (~1 L): | |
|---|---|
| Ingredient | Amount |
| $ZnCl_2$ | 40 mg |
| $FeCl_3 \cdot 6H_2O$ | 200 mg |
| $CuCl_2 \cdot 2H_2O$ | 10 mg |
| $MnCl_2 \cdot 4H_2O$ | 10 mg |
| $Na_2B_4O_7 \cdot 10H_2O$ | 10 mg |
| $(NH_4)_6Mo_7O_{24} \cdot 4H_2O$ | 10 mg |
| $H_2O$ | 1 L |

| 3. R2 Regeneration Media (~ 1 L): | |
|---|---|
| Ingredient | Amount |
| Sucrose | 103 g |
| K₂SO₄ | 0.25 g |
| Trace element solution | 2 ml |
| MgCl₂•6H₂O | 10.12 g |
| glucose | 10 g |
| L-asparagine•1H₂O | 2.0 g |
| casamino acids | 0.1 g |
| Agar | 22 g |
| Water | to 700 ml |
| The pH is adjusted to pH = 7.2 before autoclaving. After autoclaving, add: | |
| KH₂PO₄ (0.05 g/100 ml) | 100 ml |
| CaCl₂ (2.22 g/100 ml) | 100 ml |
| TES Buffer (5.73 g/100 ml, pH = 7.2) | 100 ml |

| 4. Soft Nutrient Agar (SNA, ~1 L): | |
|---|---|
| Ingredient | Amount |
| Difco Bacto Nutrient Broth | 8 g |
| Agar | 5 g |

| 5. AS1 Agar (~1 L): | |
|---|---|
| Ingredient | Amount |
| Yeast extract | 1.0 g |
| L-Alanine | 0.2 g |
| L-Arginine Free Base | 0.2 g |
| L-Asparagine | 0.5 g |
| Soluable Starch | 5.0 g |
| NaCl | 2.5 g |
| NaSO₄ | 10.0 g |
| Meer Agar | 20.0 g |

B. Transformation

Plasmid pOJ315 was used to transform Streptomyces fradiae GS33 (NRRL 18420 deposited September 23, 1988) in substantial accordance with the procedure set forth below.

Streptomyces fradiae GS33 contains the tylL mutant and therefore cannot produce the antibiotic, tylosin. A culture of S. fradiae GS33 was started from 1 ml of frozen stock into 10 ml of TSB and incubated at 30°C for about 72 hours. TSB is made at 30 g/l and is obtained from Baltimore Biological Laboratories (BBL), P.O. Box 243, Cockeysville, Maryland 21031. The culture was incubated in an air-shaker incubator at 30°C for ~30 hours. This culture was homogenized and sonicated; then, 3 ml of the culture were used to inoculate 17 ml of TSB containing 0.4% glycine. The culture was incubated in an air-shaker incubator at

15

30°C for about ~24 hours. This culture was again homogenized and sonicated; then, 3 ml of the culture were used to inoculate 17 ml of TSB containing 0.4% glycine. The culture was incubated at 30°C for about 16 hours. The culture was again homogenized and sonicated; then, the mycelial fragments were harvested and washed two times with a 10.3% sucrose solution. The mycelial fragments were resuspended in 20 ml of P media (Example 3A1) containing 1 mg/ml lysozyme, and the resulting solution was incubated at room temperature for about one to one-and-one-half hours. During this protoplasting step, the cells were pipetted up and down to disperse clumps. The protoplasts were collected and washed two times with P medium. The protoplasts were then suspended in 2 ml of P medium. This process usually generates about 2 to 5 × $10^7$ protoplasts per 200 μl of solution.

Approximately 200 μl of the protoplast solution were used per transformation. About 1 μg of the transforming DNA, in 10 μl of either ligation or TE buffer, was added to 50 μl of a 1 mg/ml solution of heparin (Sigma Chemical Co, P.O. Box 14508, St. Louis, Missouri 63178) and mixed. The DNA solution was added to the protoplasts; then, about 0.9 ml of 55% polyethylene glycol 1000 (Sigma) in P media were added to and mixed with the protoplasts. The cell-DNA mixture was vortexed and then plated onto R2 medium (Example 3A3); each plate was inoculated with about 0.1 ml of cells mixed with ~3 ml of R2-modified soft agar (103 g sucrose, 0.5% agar 10.12 g $MgCl_2$, 2.22 g $CaCl_2$, and 5.72 g TES at pH = 7.2 per liter). The plates were incubated at 30°C overnight (~16 hours) and then overlaid with 3 ml of R2-modified soft agar containing enough apramycin to give a final concentration, after diffusion, of 25 μg/ml. The plates were then incubated for about four days at 30°C, when colonies became visible to the unaided eye.

The transformation efficiency of this protocol can be increased by first transforming the plasmids into Streptomyces fradiae M1, re-isolating the plasmids and then transforming into the mutant S. fradiae strain. Streptomyces fradiae M1 was first disclosed in Baltz, Journal of General Microbiology, 1978, 107:93-103.


Example 4



Assays of S. fradiae GS33 Transformants


A. Plate-Plug Assay

Streptomyces fradiae GS33 (NRRL 18420)/pOJ315 transformants were patched from the R2-agar regeneration plates to plates containing AS1 agar (Example 3A5) and 25 μg/ml apramycin and incubated at 37°C for 2-3 days until the colonies were 5-10 millimeters in diameter. The colonies were then plugged and the plugs transferred, using a sterile transfer tube (Spectrum Medical Industrial, Inc., Los Angeles, CA 90054) to trypticase soy agar (TSA) plates, which had been previously overlayed with soft-agar nutrient broth (Difco Laboratories, Detroit, MI 48232) containing Micrococcus luteus X160 (ATCC 9341). The plates were incubated at 37°C for 16-24 hours. Micrococcus luteus (ATCC 9341) is sensitive to tylosin and resistant to apramycin. Consequently, this M. luteus strain cannot grow around a plug which contains Streotomyces that are producing tylosin. In the plate-plug assay done with S. fradiae wild-type cells (NRRL 2702)and S. fradiae GS33/pOJ315, there were clear zones of inhibition of M. luteus growth around the plugs. These zones, about 20 to 25 mM in size, of inhibition, which were absent around the S. fradiae GS33 plugs, indicated the transformants were producing tylosin.


B. Bioautography

Several plugs were prepared from the plates containing Streptomyces fradiae GS33/pOJ315 used in Example 4A. These plugs were placed onto a thin-layer chromatography plate (Merck, P.O. Box 2000, Rahway, New Jersey 07065, pre-coated silica gel #60 F-254) next to a sample of tylosin standard. The plugs were left on the plate for a time sufficient for diffusion to occur; then, the plate was developed by ascending liquid chromatography in 95:5:5 ethylacetate:diethylamine:methanol. The developed chromatograms were dried thoroughly in a fume hood for at least two hours. The chromatograms were then placed face down on Micrococcus luteus X160-seeded TSA plates for ~15 minutes. The chromatograms were removed from the plates, and the plates were incubated at 37° for 16-24 hours.

The chromatograms for the plugs prepared from Streptomyces fradiae GS33/pOJ315 transformants produced zones of inhibition resulting from substances on the chromatogram that comigrated with the tylosin standard.

## Example 5

### Construction of Plasmid pOJ182

Plasmid pOJ174 can be obtained from the NRRL in E. coli K12 SF8/pOJ174, deposited June 4, 1987, under the accession number NRRL B-18221. Plasmid pOJ174 can be isolated from E. coli K12 SF8/pOJ174 in substantial accordance with the teaching of Example 1. A restriction site and function map of plasmid pOJ174 is presented in Figure 5 of the accompanying drawings.

To prepare plasmid pOJ182, about 1 µg of plasmid pOJ174 was first digested with restriction enzyme EcoRI, in substantial accordance with the teaching of Example 2. The EcoRI-digested plasmid pOJ174 fragments were then digested with restriction enzyme AhaIII in substantial accordance with the teaching of Example 2. After precipitation and resuspension, about 4 µg of EcoRI and AhaIII digested plasmid pOJ174 fragments were ligated into EcoRI-digested, dephosphoxylated plasmid pOJ160, in substantial accordance with the teaching of Example 2. The ligation mixture was transformed into E. coli JM109 (Stratagene) in substantial accordance with the teaching of Example 2. Subsequent plasmid isolation and restriction enzyme analysis revealed plasmids which comprise an ~10 kb insert which contains the carM gene. These plasmids were designated plasmids pOJ182 and pOJ182A, which differ only in the orientation of the ~10 kb EcoRI restriction fragment. A restriction site and function map of plasmid pOJ182 is presented in Figure 6 of the accompanying drawings. Alternatively, the ~10 kb EcoRI carM-containing restriction fragment used to construct plasmid pOJ182 can be isolated from plasmid pOJ220.

## Example 6

### Construction of Plasmid pOJ226

To prepare plasmid pOJ226, approximately 2 µg of plasmid pOJ182 were digested with restriction enzyme PstI in substantial accordance with the teaching of Example 2, except 10X PstI restriction buffer (100 mM Tris-HCl, pH 7.5; 1 M NaCl; 1 mg/ml BSA and 100 mM MgCl₂) and restriction enzyme PstI were used. This digestion cuts plasmid pOJ182 at two sites; once in the S. thermotolerans insert and once in the polylinker region of plasmid pOJ160. The PstI-digested plasmid pOJ182 was then ligated and transformed into E. coli JM109 in substantial accordance with the teaching of Example 2. Restriction enzyme analysis of the plasmids isolated from transformants reveals plasmid pOJ226, which is a PstI deletion of plasmid pOJ182 comprising the carM gene on an ~2 kb restriction fragment. A restriction site and function map of plasmid pOJ226 is presented in Figure 7 of the accompanying drawings.

## Example 7

### Transformation of Streptomyces fradiae GS62 (NRRL 18421) to Produce S. fradiae GS62/pOJ182 and S. fradiae GS62/pOJ226

Plasmid pOJ182 and plasmid pOJ226 were individually used to transform Streptomyces fradiae GS62 (NRRL 18421 deposited September 23, 1988) in substantial accordance with the teaching of Example 3.

## Example 8

17

Assays of S. fradiae GS62 Transformants

The Plate-Plug assay of Example 4A was used to confirm that S. fradiae GS62/pOJ182 and S. fradiae GS62/pOJ226 were all producing antibiotic. In addition, the Bioautography Assay of Example 4B was also used to confirm that each of the selected transformants produced an antibiotic which co-migrated with the tylosin standard. NMR and Mass Spectroscopy revealed that the antibiotic produced by the transformed strains was tylosin.

**Claims**

1. A method for increasing the amount of a sugar biosynthesis enzyme in an organism, said method comprising:
a) transforming said organism with a recombinant DNA vector that codes for expression of the carL gene product, and
b) culturing said organism transformed in step (a) under conditions that allow for gene expression.

2. The method of Claim 1, wherein the organism is Streptomyces, Streptomyces fradiae or Streptomyces fradiae GS33 (NRRL 18420).

3. The method of Claim 1 or 2 wherein the recombinant DNA vector is plasmid pOJ315, as shown in Figure 3.

4. A recombinant DNA compound that comprises the carL gene.

5. The DNA compound of Claim 4 that is the ~20 kb EcoRI restriction fragment of plasmid pOJ315.

6. The DNA compound of Claim 4 that is plasmid pOJ220, as shown in Figure 4, or pOJ315.

7. A recombinant DNA host cell transformed with a vector that comprises the carL gene.

8. The recombinant DNA host cell of Claim 7 that is E. coli K12 SF8/pOJ220 (NRRL B-18222) or Streptomyces fradiae GS33 (NRRL 18420)/pOJ315.

9. A method for producing a hybrid antibiotic in a host cell, said method comprising:
a) transforming the host cell with a recombinant DNA vector that drives expression of the carL gene product, and
b) culturing the transformed host cell under conditions suitable for producing the hybrid antibiotic.

10. The method of Claim 9 wherein said host cell is a macrolide-producing host cell.

11. A method for increasing the amount of a sugar biosynthesis enzyme in an organism, said method comprising:
a) transforming said organism with a recombinant DNA vector that codes for expression of the carM gene product, and
b) culturing said organism transformed in step (a) under conditions that allow for gene expression.

12. The method of Claim 11 wherein the organism is Streptomyces, Streptomyces fradiae or Streptomyces fradiae GS62 (NRRL 18421).

13. The method of Claim 11 or 12 wherein the recombinant DNA vector is plasmid pOJ182, as shown in Figure 6 or pOJ226, as shown in Figure 7.

14. A recombinant DNA compound that comprises the carM gene.

15. The DNA compound of Claim 14 that is the ~2 kb PstI-EcoRI restriction fragment of plasmid pOJ226.

16. The DNA compound of Claim 14 that is plasmid pOJ174, as shown in Figure 5, or pOJ226.

17. A recombinant DNA host cell transformed with a vector that comprises the carM gene.

18. The recombinant DNA host cell of Claim 17 that is E. coli K12 SF8/pOJ174 (NRRL B-18221), Streptomyces fradiae GS62 (NRRL 18421)/pOJ182 or Streptomyces fradiae GS62 (NRRL 18421)/pOJ226.

19. A method for producing a hybrid antibiotic in a host cell, said method comprising:
a) transforming the host cell with a recombinant DNA vector that drives expression of the carM gene product, and
b) culturing the transformed host cell under conditions suitable for producing the hybrid antibiotic.

20. The method of Claim 19 wherein said host cell is a macrolide-producing host cell.

Claims for the following Contracting State: GR

1. A method for increasing the amount of a sugar biosynthesis enzyme in an organism, said method comprising:
a) transforming said organism with a recombinant DNA vector that codes for expression of the carL gene product, and

b) culturing said organism transformed in step (a) under conditions that allow for gene expression.

2. The method of Claim 1, wherein the organism is Streptomyces, Streptomyces fradiae or Streptomyces fradiae GS33 (NRRL 18420).

3. The method of Claim 1 or 2 wherein the recombinant DNA vector is plasmid pOJ315, as shown in Figure 3.

4. A recombinant DNA compound that comprises the carL gene.

5. The DNA compound of Claim 4 that is the ~20 kb EcoRI restriction fragment of plasmid pOJ315.

6. The DNA compound of Claim 4 that is plasmid pOJ220, as shown in Figure 4, or pOJ315.

7. A recombinant DNA host cell transformed with a vector that comprises the carL gene.

8. The recombinant DNA host cell of Claim 7 that is E. coli K12 SF8/pOJ220 (NRRL B-18222) or Streptomyces fradiae GS33 (NRRL 18420)/pOJ315.

9. A method for producing a hybrid antibiotic in a host cell, said method comprising:

a) transforming the host cell with a recombinant DNA vector that drives expression of the carL gene product, and

b) culturing the transformed host cell under conditions suitable for producing the hybrid antibiotic.

10. The method of Claim 9 wherein said host cell is a macrolide-producing host cell.

11. A method for increasing the amount of a sugar biosynthesis enzyme in an organism, said method comprising:

a) transforming said organism with a recombinant DNA vector that codes for expression of the carM gene product, and

b) culturing said organism transformed in step (a) under conditions that allow for gene expression.

12. The method of Claim 11 wherein the organism is Streptomyces, Streptomyces fradiae or Streptomyces fradiae GS62 (NRRL 18421).

13. The method of Claim 11 or 12 wherein the recombinant DNA vector is plasmid pOJ182, as shown in Figure 6 or pOJ226, as shown in Figure 7.

14. A recombinant DNA compound that comprises the carM gene.

15. The DNA compound of Claim 14 that is the ~2 kb PstI-EcoRI restriction fragment of plasmid pOJ226.

16. The DNA compound of Claim 14 that is plasmid pOJ174, as shown in Figure 5, or pOJ226.

17. A recombinant DNA host cell transformed with a vector that comprises the carM gene.

18. The recombinant DNA host cell of Claim 17 that is E. coli K12 SF8/pOJ174 (NRRL B-18221), Streptomyces fradiae GS62 (NRRL 18421)/pOJ182 or Streptomyces fradiae GS62 (NRRL 18421)/pOJ226.

19. A method for producing a hybrid antibiotic in a host cell, said method comprising:

a) transforming the host cell with a recombinant DNA vector that drives expression of the carM gene product, and

b) culturing the transformed host cell under conditions suitable for producing the hybrid antibiotic.

20. The method of Claim 19 wherein said host cell is a macrolide-producing host cell.


Claims for the following Contracting State: ES

1. A method for increasing the amount of a sugar biosynthesis enzyme in an organism, said method comprising:

a) transforming said organism with a recombinant DNA vector that codes for expression of the carL gene product, and

b) culturing said organism transformed in step (a) under conditions that allow for gene expression.

2. The method of Claim 1, wherein the organism is Streptomyces , Streptomyces fradiae or Streptomyces fradiae GS33 (NRRL 18420).

3. The method of Claim 1 or 2 wherein the recombinant DNA vector is plasmid pOJ315, as shown in Figure 3.

4. The method of Claim 1, 2 or 3 wherein the organism cultured in step (b) is Streptomyces fradiae GS33 (NRRL 18420)/pOJ315.

5. The method of Claim 1, 2 or 3 wherein the organism cultured in step (b) is Streptomyces fradiae GS33 (NRRL 18420)/pOJ220.

6. A method for producing a hybrid antibiotic in a host cell, said method comprising:

a) transforming the host cell with a recombinant DNA vector that drives expression of the carL gene product, and

b) culturing the transformed host cell under conditions suitable for producing the hybrid antibiotic.

7. The method of Claim 6 wherein said host cell is a macrolide-producing host cell.

8. The method of Claim 7 wherein the macrolide-producing host cell is a Streptomyces host cell.

9. A method for increasing the amount of a sugar biosynthesis enzyme in an organism, said method comprising:

a) transforming said organism with a recombinant DNA vector that codes for expression of the carM gene product, and

b) culturing said organism transformed in step (a) under conditions that allow for gene expression.

10. The method of Claim 9 wherein the organism is Streptomyces, Streptomyces fradiae or Streptomyces fradiae GS62 (NRRL 18421).

11. The method of Claim 9 or 10 wherein the recombinant DNA vector is plasmid pOJ182, as shown in Figure 6 or pOJ226, as shown in Figure 7.

12. The method of Claim 11, 12 or 13 wherein the organism cultured in step (b) is Streptomyces fradiae GS62 (NRRL 18421)/pOJ174.

13. The method of Claim 11, 12 or 13 wherein the organism cultured in step (b) is Streptomyces fradiae GS62 (NRRL 18421)/pOJ182.

14. The method of Claim 11, 12 or 13 wherein the organism cultured in step (b) is Streptomyces fradiae GS62 (NRRL 18421)/pOJ226.

15. A method for producing a hybrid antibiotic in a host cell, said method comprising:

a) transforming the host cell with a recombinant DNA vector that drives expression of the carM gene product, and

b) culturing the transformed host cell under conditions suitable for producing the hybrid antibiotic.

16. The method of Claim 15 wherein said host cell is a macrolide-producing host cell.

17. The method of Claim 16 wherein the macrolide-producing host cell is a Streptomyces host cell

# FIG. I
## Restriction Site and Function Map of Plasmid pOJ171
### (~45 kb)

# FIG.2
# Restriction Site and Function Map of
# Plasmid pOJ160
## (~7 kb)

# FIG.3
## Restriction Site and Function Map of
## Plasmid pOJ315
### (~27 kb)

# FIG.4
## Restriction Site and Function Map of
## Plasmid pOJ220
### (~45 kb)

# FIG.5
## Restriction Site and Function Map of Plasmid pOJ174
### (~45 kb)

# FIG. 6
# Restriction Site and Function Map of
# Plasmid pOJ182
## (~17 kb)

# FIG.7
# Restriction Site and Function Map of
# Plasmid pOJ226
## (~9 kb)

# FIG.8
## Function Map of the
## *Streptomyces thermotolerans* Chromosome of
## the Biosynthetic Gene Cluster

←————————100 kilobases————————→

MLS$^R$ (carB)                                MLS$^R$

carE  carG          carL          carM carA

Lactone    Sugar      Sugar

Acylase

├————————171————————┤ ├————————174————————┤
├————————220————————┤

182

315

226